# EUROPÄISCHE PATENTSCHRIFT

(11) **EP 0 482 619 B1**
(45) Veröffentlichungstag und Bekanntmachung des Hinweises auf die Patenterteilung: **31.05.1995**
(21) Anmeldenummer: 91118092.5
(22) Anmeldetag: 23.10.1991
(51) Int. Cl.: C12Q 1/16, C12Q 1/04

(54) **Vorrichtung und Screening-Verfahren zum Nachweis von Mikroorganismen, die niedermolekulare flüchtige Stoffwechselprodukte bilden**
Device and screening method to detect micro-organisms which form low molecular volatile metabolites
Dispositif et méthode de screening pour détecter des microorganismes produisant des métabolites volatils de masse moléculaire faible

(30) Priorität: 23.10.1990 CH 3391/90
(43) Veröffentlichungstag der Anmeldung: 29.04.1992
(73) Patentinhaber: LONZA AG, CH-3945 Gampel/Wallis (CH)
(72) Erfinder: Kulla, Hans, Dr., Visperterminen (Kanton Wallis) (CH)
(74) Vertreter: Weinhold, Peter, Dr.

(56) Entgegenhaltungen:
- EP-A- 0 124 285
- APPLIED AND ENVIRONMENTAL MICROBIOLOGY Bd. 35, Nr. 6, Juni 1978,WASHINGTON DC USA Seiten 1215 - 1218; T.A. MCMEEKIN ET AL: 'Detection ofvolatile sulfide-producing bacteria isolated from poultry-processing plants.'
- PATENT ABSTRACTS OF JAPAN vol. 9, no. 309 (C-318)(2032)5. Dezember 1985 & JP-A-60 149 382 ( NITSUSHIN DENKI KK ) 6. August 1985

## Beschreibung

Die Erfindung betrifft eine neue Vorrichtung sowie ein neues Screening-Verfahren zum Nachweis von Mikroorganismen, die bei einem mikrobiologischen Prozess aus einem radioaktiv markierten Substrat ein radioaktiv markiertes niedermolekulares flüchtiges Stoffwechselprodukt bilden. Dabei werden die Mikroorganismen nachgewiesen, die ein radioaktiv markiertes Substrat in ein gewünschtes Produkt umsetzen, wobei ein radioaktiv markiertes niedermolekulares flüchtiges Stoffwechselprodukt anfällt.

Generell ist bekannt, dass man die bei einem mikrobiologischen bzw. biochemischen Prozess gebildeten Stoffwechselprodukte derart nachweisen kann, dass man eine radioaktiv markierte Verbindung zu einer Zellsuspension zufügt, das Ganze für eine bestimmte Zeit inkubiert und dann nach Abtrennen der Zellen das gebildete radioaktiv markierte Stoffwechselprodukt beispielsweise mittels Flüssigkeitsszintillationsmessung bestimmt (Firmin, J.L. und Gray D.O., Biochem.J., 1976, 158, S.223-229).

Ein grosser Nachteil dieser allgemein gängigen Nachweismethode besteht einerseits darin, dass sie umständlich und zeitraubend ist. Andererseits eignet sich diese Methode nicht zur Selektion von Mikroorganismen mit bestimmten Stoffwechselleistungen, da unabhängig vom Molekulargewicht alle gebildeten radioaktiv markierten Stoffwechselprodukte nachgewiesen werden.

Desweiteren ist aus der EP-A 124 285 eine Nachweismethode für Mikroorganismen in medizinisch zu untersuchenden Proben bekannt.
Auch diese Methode hat den Nachteil, dass unabhängig vom Molekulargewicht unspezifisch sämtliche radioaktiv markierten Stoffwechselprodukte nachgewiesen werden. Damit stellt auch diese Methode kein Screening-Verfahren für Mikroorganismen mit bestimmten Stoffwechselprodukten dar.

Aufgabe der vorliegenden Erfindung war es, ein empfindliches Screening-Verfahren für Mikroorganismen zu entwickeln, mit dem quantitativ alle Mikroorganismen nachgewiesen werden können, die aus einem bestimmten Substrat niedermolekulare flüchtige Stoffwechselprodukte bilden.

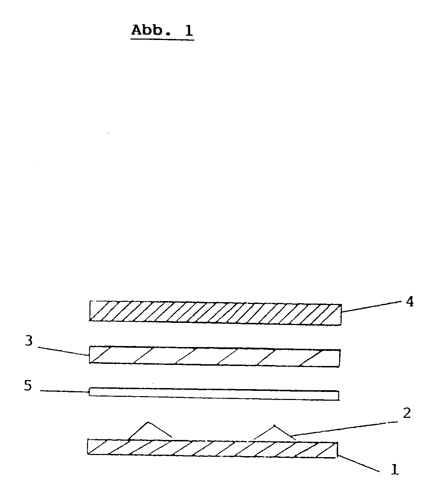

Diese Aufgabe wurde durch eine neue Vorrichtung nach Patentanspruch 1 und damit durch ein neues Screening-Verfahren gelöst. Abbildung 1 zeigt schematisch die erfindungsgemässe Vorrichtung. Bei dem Screening-Verfahren werden die Mikroorganismen nachgewiesen, die bei einem mikrobiologischen Prozess niedermolekulare flüchtige Stoffwechselprodukte bilden, wobei der Nachweis so erfolgt, dass ein radioaktiv markiertes Substrat durch den Mikroorganismus in das radioaktiv markierte niedermolekulare flüchtige Stoffwechselprodukt umgesetzt wird und dann das radioaktiv markierte Stoffwechselprodukt durch einen semipermeablen Filter, (3) in Abb. 1, von höhermolekularen nichtflüchtigen radioaktiv markierten Produkten oder Edukten abgetrennt, an einer Adsorptionsplatte (4) adsorbiert und dort über die Bestimmung der Radioaktivität nachgewiesen wird.

Als radioaktiv markiertes Stoffwechselprodukt wird ein niedermolekulares flüchtiges Stoffwechselprodukt nachgewiesen. Als niedermolekulare Stoffwechselprodukte werden Stoffwechselprodukte mit einem Molekulargewicht von nicht höher als 500 bezeichnet. Vorzugsweise ist das Molekulargewicht der Stoffwechselprodukte nicht höher als 250.

Unter flüchtigen Stoffwechselprodukten sind die durch Mikroorganismen gebildeten Stoffwechselprodukte zu verstehen, die in der Lage sind, unter dem Einfluß der Adsorptionsplatte (4) und den angegebenen Versuchsbedingungen durch den semipermeablen Filter (3) in gasförmigem Zustand hindurch zu diffundieren.

Semipermeable Filter (3) sind somit sämtliche Filter, insbesondere der angegebenen Porengröße, die unter den Versuchsbedingungen einen Durchgang allein dieser flüchtigen Stoffwechselprodukte in ausreichendem Maße gewährleisten, den Durchgang höhermolekularer nichtflüchtiger Verbindungen jedoch nicht gestatten.

Als Substrate werden radioaktiv markierte Substrate eingesetzt, die beispielsweise mit den Isotopen ¹⁴C, ³H, ³⁵S und ³²P oder mit anderen biorelevanten Isotopen markiert sind. Vorzugsweise sind die Substrate mit den Isotopen ³H und ¹⁴C markiert. Die spezifische Radioaktivität der radioaktiv markierten Substrate liegt üblicherweise in einem Bereich zwischen 1 mCi/mmol bis 20 Ci/mmol.

Die Substrate sind zweckmässig so radioaktiv markiert, dass als niedermolekulares flüchtiges Stoffwechselprodukt entweder ein ³H-markiertes oder ein ¹⁴C-markiertes Stoffwechselprodukt gebildet wird, wie beispielsweise ³H-markiertes Wasser, ¹⁴C-markierte Produkte wie beispielsweise Kohlendioxid, Ethanol, Essigsäure, Ameisensäure, Aceton, Butanol, Acetoin oder andere Stoffwechselprodukte. Insbesondere werden ³H-markierte Substrate angewendet, bei denen ³H-markiertes Wasser gebildet wird, und ¹⁴C-markierte Substrate, bei denen ¹⁴CO₂ gebildet wird.

Die radioaktiv markierten flüchtigen Stoffwechselverbindungen können mit den in der Fachwelt üblichen Methoden, wie beispielsweise Autoradiographie, Flüssigkeitsszintillationsmessung, nachgewiesen werden, vorzugsweise wird das Stoffwechselprodukt mittels Autoradiographie bestimmt.

Das Screening-Verfahren wird in einer Vorrichtung gemäss Abbildung 1 durchgeführt, die aus einer Membran (1), die die wachsenden Mikroorganismen (2) trägt, besteht, auf der ein semipermeabler Filter (3), der für das flüchtige Stoffwechselprodukt permeabel, für höhermolekulare nichtflüchtige Produkte oder Edukte jedoch nicht durchlässig ist, und eine Adsorptionsplatte (4) aufgebracht sind.

Zweckmässig wachsen die Mikroorganismen-Kolonien (2) auf einer Membran (1) auf, die auf Standard-Agarmedium aufgebracht wurde, wie beispielsweise auf Plate-Count-Agar (Firma Difco Laboratories, USA).

Wenn die Mikroorganismen-Kolonien einen Durchmesser von 0,1 bis 5 mm, vorzugsweise von 0,5 bis 2 mm, erreicht haben, werden sie mit einer Inkubationslösung, die das radioaktiv markierte Substrat enthält, überschichtet. Um Konvektionen in der Inkubationslösung zu minimieren, kann die Zugabe von Viskositätsbildner, wie Agar, erfolgen.

Geeignet sind prinzipiell alle Membranen, die für die verwendeten Nährlösungen durchlässig sind. Vorzugsweise wird als Membran (1) ein Membranfilter, insbesondere ein hydrophiler Sterilfilter mit einer Porengrösse von 0,01 bis 2 »m aufgebracht. Beispielsweise können Sterilfilter aus Cellulosenitrat, Celluloseacetat, regenerierter Cellulose, Nylon oder andere standardmässige Sterilfilter, angewendet werden.

Beim Nachweis von ³H₂O kann es zweckmässig sein, die bewachsene Membran (1) vor der Inkubation vom verfestigten Nährmedium abzuheben und zu transferieren, um eine übermässige Verdünnung des ³H₂O zu verhindern.

Nach Überschichtung der Mikroorganismen mit der Inkubationslösung wird ein semipermeabler hydrophober oder ein hydrophobierter Filter (3) mit einer Porengrösse, zweckmässig von 0,02 bis 2 »m aufgelegt, der für das flüchtige Stoffwechselprodukt permeabel, für höhermolekulare nichtflüchtige Produkte oder Edukte jedoch nicht durchlässig ist.

Die Auswahl des semipermeablen Filters ist nicht kritisch und dem Fachmann anhand der vorliegenden Beschreibung ohne weiteres möglich. Vorzugsweise wird als Material für den semipermeablen Filter (3) entweder Goretex® (Polytetrafluorethylen PTFE mit einer Porengrösse von 2 bis 0,02 »m auf Polyester-Stützgewebe), oder ein hydrophobierter Glasfaser-Filter, insbesondere ein silanisierter Glasfaser-Filter, oder Silikon-Membranen mit einer Schichtdicke von 0,1 bis 1 mm, angewendet. Es kann auch aus hydrophoben, pulverförmigen Materialien, wie z.B. mit Aerosil R972 (Firma Degussa, BRD), eine semipermeable Schicht angelegt werden.

Um ein Vermischen der Kolonien zu verhindern, wird zweckmässig ein Stützgewebe (5) zwischen Membran (1) und semipermeablen Filter (3) eingelegt. Als Stützgewebe können beispielsweise Baumwollgewebe oder Netze, wie Gaze, verwendet werden. Es kann aber auch eine weitere Membran aufgebracht werden.

Auf den semipermeablen Filter (3) wird dann eine Adsorptionsplatte (4) aufgebracht, wobei vorzugsweise zum Nachweis von ³H₂O als Adsorptionsplatte eine mit Molekularsieb oder mit Kieselgel beschichtete Glasplatte angewendet wird. Zum Nachweis von ¹⁴CO₂ wird vorzugsweise eine mit Kalk beschichtete Glasplatte angewendet.

Insbesondere wird die Glasplatte mit einem Molekularsieb mit einer Porengrösse von 3-4 Å oder mit einem Kieselgel beschichtet, wobei die Schichtdicke zwischen 0,1 und 5 mm liegt. Es können jedoch auch andere Adsorbentien als Material für die Adsorptionsplatte geeignet sein, wie beispielsweise Gips oder Aluminiumoxid.
Zum Verfestigen der Adsorptionsplatten kann es zweckmässig sein, Bindemittel, wie beispielsweise derivatisierte Cellulose, Stärke, Zement, Wasserglas, Glasfasern oder andere zur Herstellung von Dünnschichtchromatographie-Platten übliche Bindemittel, zu verwenden.
Danach wird die ganze Vorrichtung abhängig vom Material des semipermeablen Filters (3) 1 bis 12 Stunden inkubiert, bei einer zweckmässigen Temperatur von 5 bis 110°C, wobei die in der Fachwelt üblichen Inkubationsmedien angewendet werden können. Durch Auflegen eines Gewichts wird diese Vorrichtung beispielsweise auf 5 mm zusammengedrückt. Die Länge der Vorrichtung kann beispielsweise 5 cm betragen.
Vorzugsweise wird die Vorrichtung, wenn Silikon-Membranen als semipermeabler Filter (3) angewendet werden, bis zu 3 Stunden inkubiert, bei Goretex oder den hydrophobierten Glasfaser-Filtern vorzugsweise bis zu 1 Stunde inkubiert. Ueblicherweise ist die Inkubationstemperatur von den Temperatureigenschaften der Mikroorganismen abhängig und kann von einem Fachmann leicht ermittelt werden. Wenn beispielsweise mesophile Mirkoorganismen nachgewiesen werden, liegt die Inkubationstemperatur vorzugsweise zwischen 30 und 37°C.

Während der Inkubation setzen die Mikroorganismen das radioaktiv markierte Substrat zu einem gewünschten Produkt um, wobei ein radioaktiv markiertes niedermolekulares flüchtiges Stoffwechselprodukt gebildet wird. Dieses flüchtige Stoffwechselprodukt diffundiert durch den semipermeablen Filter (3) und adsorbiert auf die Adsorptionsplatte (4).

Nach der Inkubation wird die Adsorptionsplatte abgenommen und kann für den Nachweis des flüchtigen Stoffwechselproduktes mit den für die Fachwelt üblichen Szintillatoren, wie z.B. Enhance® (Firma DuPont, USA), behandelt werden. Es können jedoch auch andere Szintillationslösungen oder die von B.R. Bochner und B.N. Arnes in Anal. Biochem. 131, S. 510-515 (1984) beschriebene angewendet werden.

Das erfindungsgemässe Verfahren stellt eine empfindliche Methode dar, um Mikroorganismen nachzuweisen, die ein radioaktiv markiertes Substrat in ein gewünschtes Produkt umsetzen, wobei ein radioaktiv markiertes niedermolekulares flüchtiges Stoffwechselprodukt anfällt. Diese Mikroorganismen können dann aufgrund dieser Nachweismethode besser selektioniert werden. Im Unterschied zu herkömmlichen Verfahren funktioniert dieses Verfahren auch mit Kolonien von Mikroorganismen, wie sie auf der Oberfläche verfestigter Nährmedien wachsen, wodurch die Kapazität erheblich erhöht wird.

Die Vorrichtung eignet sich auch zum Nachweis von Mikroorganismen, die befähigt sind, Methylgruppen in Verbindungen zum entsprechenden sprechenden Alkohol, Aldehyd oder zur entsprechenden Carbonsäure umzusetzen. Der Nachweis erfolgt dabei indirekt, indem während der Oxidation ebenfalls gebildete niedermolekulare flüchtige Stoffwechselprodukte nachgewiesen werden.
Beispielsweise kann die Vorrichtung zum Nachweis von Mikroorganismen verwendet werden, die befähigt sind, die Methylgruppen von 5-Methyl-2-chlorpyridin zur entsprechenden Säure zu oxidieren, wobei das dabei gebildete ³H₂O auf der Adsorptionsplatte nachgewiesen wird.

### Beispiel 1

Alcaligenes entrophus (DSM 428) und Pseudomonas aeruginosa (DSM 50071) wurden getrennt in Nutrient-Broth (Firma Oxoid, USA) bei 30°C über Nacht auf der Schüttelmaschine gezüchtet. Dann wurde 1 Teil der Pseudomonas-Kultur mit 999 Teilen der Alcaligenes-Kultur gemischt und 0,1 ml dieser Mischung in der Verdünnung 10⁻⁴ auf einen auf Plate-Count-Agar (PCA, Firma Difco, USA) aufliegenden Cellulosenitratfilter (1) mit 0,2 »m Porengrösse und 5 cm Durchmesser (Firma Sartorius, BRD) ausplatiert. Die Zellen wuchsen über Nacht zu etwa 1000 Kolonien heran mit einem Durchmesser von 0,5 bis 1 mm. Der die Kolonien tragende Filter wurde vom PCA-Medium (Firma Difco, USA) abgehoben und mit der Kolonie nach oben in eine Petrischale gelegt. Die Membran (1) mit den Kolonien wurde mit 0,18 ml radioaktiver Lösung folgender Zusammensetzung durchtränkt:
5 g/l Agar (geschmolzen)
5 g/l Trypton (Firma Difco, USA)
2,5 g/l Hefextrakt (" " " )
2 mM D-[6-³H(N)]-Glukose 0,1 Ci/mmol (Firma NEN/DuPont, USA)
Es wurde sofort ein zweiter, durch Auflegen auf Plate-Count-Agar angefeuchteter Cellulosenitratfilter (5) auf die Kolonie aufgelegt. Darauf wurde sofort ein hydrophober TE35-Filter (3) (PTFE auf Polyestern, Firma Schleicher u. Schuell, BRD) mit 0,2 »m Porengrösse und einem Durchmesser von 8 cm aufgelegt. Sofort wurde mit der Adsorptionsschicht nach unten die wasserspezifische Adsorptionsplatte (4) aufgelegt, die wie folgt hergestellt wurde:
8g pulverförmiges (Korngrösse 2-3 »m) Molekularsieb 4Å (Firma Aldrich, BRD) wurde in 7 ml Wasser aufgeschlämmt und 0,1 g Glasfaser zugegeben.
Die Glasfaser bestand aus kleingeriebenem GF/D-Filter (GF/D: Glasfaserfilter Sorte D der Firma Whatman, USA). Danach wurde im Vakuum entgast.
Die breiige Masse wurde auf eine 6x6 cm grosse, aufgerauhte Glasplatte gegossen, dann bei 70°C 1 Stunde getrocknet und danach 4 Stunden bei 150°C im Hochvakuum aktiviert. Die resultierende Schichtdicke war ca. 2 mm. Die Platten wurden über Phosphorpentoxid gelagert.

Die ganze Vorrichtung wurde durch Aufsetzen eines Stempels von 450 g Gewicht auf ca. 5 mm zusammengedrückt. Danach wurde 1 Stunde bei 30°C inkubiert. Danach wurde die MolekularsiebPlatte abgenommen und sofort mit Enhance^{®} (Firma DuPont, USA) besprüht. Anschliessend wurde vorschriftsgemäss (R.A. Laskey, Radioisotope detection by fluorography and intensifying screens; Firma Amersham, UK, Review 23, 1984) eine Autoradioangefertigt (Exposition 2 Tage). Die vereinzelt auftretenden Pseudomonas-Kolonien waren als schwarze Flecke mit einem Durchmesser von 5 mm abgebildet, während der überwiegende Hintergrund der Alcaligenes-Kolonien nicht zu sehen war. Pseudomonas veratmet die tritierte Glukose und produziert ³H₂O, während Alcaligenes Glukose nicht verstoffwechseln kann.

### Beispiel 2

Vorgehen wie in Beispiel 1, mit folgenden Modifikationen: Radioaktives Inkubationsmedium: anstatt ³H-Glukose, wurde D-[¹⁴C(U)]-Glukose (Firma NEN/DuPont, USA) (3 Ci/mol) gewählt. Hydrophober Filter (3): Ein Glasfaser-Filter GF/A (Firma Whatman, USA) wurde durch Schmoren bei 160°C bis zur Trockene mit Repel-Silan (Firma LKB, Schweden) hydrophobisiert. Die resultierende Porengrösse beträgt 1,4 »m, die Schichtdicke 0,27 mm.
Die CO₂-spezifische Adsorptionsplatte wurde wie folgt hergestellt: Atemkalk-Plätzchen (Firma Dräger, Schweiz) wurden kleingemörsert und mit Wasser teigig angerührt und mit einer Schichtdicke von ca. 2 mm auf eine Glasplatte, 6x6 cm, aufgespachtelt und anschliessend einige Tage über NaOH-Plätzchen im Exsikkator getrocknet.
Vorgehen ansonsten wie in Beispiel 1.
Die Glukose-veratmenden Pseudomonas-Kolonien entwickelten ¹⁴CO₂ und werden als schwarze Flecke mit einem Durchmesser von 5 mm abgebildet, die Glukose-inaktiven Alcaligenes-Kolonien bleiben unsichtbar.

### Beispiel 3

Vorgehen wie in Beispiel 1, mit folgenden Modifikationen: Das Agarmedium war nicht Plate-Count-Agar sondern Kohlenstofffreies Mineralmedium (H.Kulla et al.; Arch. Microbiol. 135, S.1-7 (1983)).
Die aufliegende Membran (1) aus regenerierter Cellulose (Firma Sartorius, BRD) wurde direkt durch Platieren von Biomasse aus der aeroben Stufe einer Kläranlage beimpft. Als Kohlenstoff- und Energie-Quelle diente Xylol, das in einem Exsikkator über die Gasphase versorgt wurde. Nach einer Woche sieht man Kolonien mit einem Durchmesser von 1 bis 2 mm.

### Radioaktive Inkubationslösung:

Mineralmedium wie oben mit 0,5% Agar (geschmolzen)
5 mM 2-Cl-5-(Methyl-³H)-pyridin (10 Ci/mmol)
Hydrophober Filter (3): Silikonmembran mit Schichtdicke 0,17 mm, hergestellt aus Sylgard 186 (Firma Dow Corning, USA). Zwischen Kolonien und Silikonmembran wurde als Stützgewebe (5) eine Lage handelsüblicher medizinischer Gaze eingelegt.
Inkubationsdauer: 3 Stunden.
Wasserspezifische Adsorptionsplatte (4): PSC-Fertigplatte Kieselgel 60 ohne Fluoreszenzindikator, Schichtdicke 2 mm (Firma Merck, BRD).
Vorgehen ansonsten wie in Beispiel 1.
Kolonien, die die Methyl-Gruppe im 2-Chlor-5-methyl-pyridin oxidieren können, werden als schwarze Flecke abgebildet und können vom Cellulose-Filter mit traditionellen mikrobiologischen Methoden isoliert werden.

## Patentansprüche

1. Vorrichtung zum Nachweis von Mikroorganismen, die bei einem mikrobiologischen Prozess niedermolekulare flüchtige Stoffwechselprodukte bilden, dadurch gekennzeichnet, dass sie aus einer Membran (1), die die wachsenden Mikroorganismen (2) trägt, besteht, auf die ein semipermeabler Filter (3) und eine Adsorptionsplatte (4) aufgebracht sind, wobei der semipermeable Filter (3) für das Stoffwechselprodukt permeabel und für höhermolekulare nichtflüchtige Stoffe undurchlässig ist.

2. Vorrichtung nach Patentanspruch 1, dadurch gekennzeichnet, dass als Membran (1) ein hydrophiler Sterilfilter mit einer Porengrösse von 0,01 bis 2 »m verwendet wird.

3. Vorrichtung nach mindestens einem der Patentansprüche 1 und 2, dadurch gekennzeichnet, dass als semipermeabler Filter (3) hydrophobes oder hydrophobiertes Material mit einer Porengrösse von 0,02 bis 2 »m verwendet wird.

4. Vorrichtung nach mindestens einem der Patentansprüche 1 bis 3, dadurch gekennzeichnet, dass zwischen Membran (1) und semipermeablen Filter (3) ein Stützgewebe (5) eingelegt ist.

5. Vorrichtung nach mindestens einem der Patentansprüche 1 bis 4, dadurch gekennzeichnet, dass als Material für die Adsorptionsplatte (4) entweder ein Molekularsieb, Kieselgel, Kalk, Gips oder Aluminiumoxid verwendet wird.

6. Verwendung der Vorrichtung nach mindestens einem der Patentansprüche 1 bis 5 zum Nachweis von Mikroorganismen, die ein niedermolekulares flüchtiges Stoffwechselprodukt bilden.

7. Verwendung der Vorrichtung nach Patentanspruch 7 zum Nachweis von Mikroorganismen, die befähigt sind, methylierte Verbindungen zum entsprechenden Alkohol, Aldehyd oder zur entsprechenden Carbonsäure umzusetzen.

8. Screening-Verfahren zum Nachweis von Mikroorganismen, dadurch gekennzeichnet, dass mit der Vorrichtung nach mindestens einem der Patentansprüche 1 bis 5, die Mikroorganismen nachgewiesen werden, die bei einem mikrobiologischen Prozess niedermolekulare flüchtige Stoffwechselprodukte bilden, wobei der Nachweis so erfolgt, dass ein radioaktiv markiertes Substrat durch den Mikroorganismus zu einem radioaktiv markierten niedermolekularen flüchtigen Stoffwechselprodukt umgesetzt wird und dieses durch einen semipermeablen Filter (3) von höhermolekularen nichtflüchtigen Substanzen abgetrennt, auf eine Adsorptionsplatte (4) adsorbiert und dann bestimmt wird.

9. Verfahren nach Patentanspruch 8, dadurch gekennzeichnet, dass man als Stoffwechselprodukt entweder ein ³H-markiertes oder ein ¹⁴C-markiertes Stoffwechselprodukt nachweist.

10. Verfahren nach mindestens einem der Patentansprüche 8 und 9, dadurch gekennzeichnet, dass man das Stoffwechselprodukt mittels Autoradiographie bestimmt.

## Claims

1. Device for the detection of microorganisms which form volatile metabolites of low molecular weight during a microbiological process, characterized in that it consists of a membrane (1) which carries the growing microorganisms (2), on which are applied a semipermeable filter (3) and an adsorption sheet (4), the semipermeable filter (3) being permeable for the metabolite and impermeable for non-volatile substances of higher molecular weight.

2. Device according to claim 1, characterized in that a hydrophilic sterile filter having a pore size of 0.01 to 2 »m is used as membrane (1).

3. Device according to at least one of claims 1 and 2, characterized in that hydrophobic or hydrophobized material having a pore size of 0.02 to 2 »m is used as semipermeable filter (3).

4. Device according to at least one of claims 1 to 3, characterized in that a supporting fabric (5) is placed between membrane (1) and semipermeable filter (3).

5. Device according to at least one of claims 1 to 4, characterized in that as material for the adsorption sheet (4) either a molecular sieve, silica gel, lime, gypsum or alumina are used.

6. Use of the device according to at least one of claims 1 to 5 for the detection of microorganisms which form a volatile metabolite of low molecular weight.

7. Use of the device according to claim 7 for the detection of microorganisms capable of transforming methylated compounds to the corresponding alcohol, aldehyde or to the corresponding carboxylic acid.

8. Screening method for the detection of microorganisms, characterized in that the microorganisms forming volatile metabolites of low molecular weight during a microbiological process are detected by means of the device according to at least one of claims 1 to 5, the detection being carried out so that a radioactively labelled substrate is transformed by the microorganism to a radioactively labelled volatile metabolite of low molecular weight which is separated from the non-volatile substances of higher molecular weight by a semipermeable filter (3), is adsorbed to an adsorption sheet (4), and then is determined.

9. Method according to claim 8, characterized in that the metabolite detected is either a ³H-labelled or a ¹⁴C-labelled metabolite.

10. Method according to at least one of claims 8 and 9, characterized in that the metabolite is detected by means of radioautography.

## Revendications

1. Dispositif pour la détection de micro-organismes qui forment, lors d'un processus microbiologique, des métabolites volatils à faible masse moléculaire, caractérisé en ce qu'il consiste en une membrane (1) qui porte les microorganismes en croissance (2), sur laquelle sont appliqués un filtre semi-perméable (3) et une plaque d'adsorption (4), le filtre semi-perméable (3) étant perméable pour le métabolite et imperméable pour les substances non volatiles à masse moléculaire élevée.

2. Dispositif selon la revendication 1, caractérisé en ce qu'en tant que membrane (1), on utilise un filtre stérile hydrophile avec une dimension de pore de 0,01 jusqu'à 2 »m.

3. Dispositif selon au moins l'une des revendications 1 et 2, caractérisé en ce qu'en tant que filtre semiperméable (3), on utilise un matériau hydrophobe ou hydrophobé avec une dimension de pore de 0,02 jusqu'à 2 »m.

4. Dispositif selon au moins l'une des revendications 1 à 3, caractérisé en ce qu'entre la membrane (1) et le filtre semi-perméable (3), est intercalé un tissu de support (5).

5. Dispositif selon au moins l'une des revendications 1 à 4, caractérisé en ce qu'en tant que matériau pour la plaque d'adsorption (4), on utilise soit un tamis moléculaire, du gel de silice, de la chaux, du plâtre ou de l'oxyde d'aluminium.

6. Utilisation du dispositif selon au moins l'une des revendications 1 à 5, pour détecter la présence de micro-organismes qui forment un métabolite volatil à faible masse moléculaire.

7. Utilisation du dispositif selon la revendication 7, pour détecter des micro-organismes qui sont capables de transformer des composés méthylés en l'alcool correspondant, aldéhyde ou en l'acide carboxylique correspondant.

8. Procédé de criblage pour détecter des microorganismes, caractérisé en ce qu'avec le dispositif selon au moins l'une des revendications 1 à 5, on détecte les microorganismes qui, au cours d'un processus microbiologique, forment des métabolites volatils à faible masse moléculaire, la détection s'effectuant de telle sorte qu'un substrat marqué radioactivement est transformé par le micro-organisme en un métabolite volatil à faible masse moléculaire marqué radioactivement, celui-ci étant séparé par un filtre semiperméable (3) des substances non volatiles à masse moléculaire élevée, adsorbé sur une plaque d'adsorption (4) puis déterminé.

9. Procédé selon la revendication 8, caractérisé en ce qu'en tant que métabolite, on détecte soit un métabolite marqué ³H ou un métabolite marqué ¹⁴C.

10. Procédé selon au moins l'une des revendications 8 et 9, caractérisé en ce que l'on détermine le métabolite au moyen d'autoradiographie.
